# EUROPÄISCHE PATENTSCHRIFT

(11) **EP 1 312 313 B1**
(45) Veröffentlichungstag und Bekanntmachung des Hinweises auf die Patenterteilung: **10.01.2007**
(21) Anmeldenummer: 02024351.5
(22) Anmeldetag: 02.11.2002
(51) Int. Cl.: A61B 17/28, A61B 17/32

(54) **Medizinische Zange**
Surgical forceps
Forceps chirurgicaux

(30) Priorität: 19.11.2001 DE 10156313
(43) Veröffentlichungstag der Anmeldung: 21.05.2003
(73) Patentinhaber: Richard Wolf GmbH, 75438 Knittlingen (DE)
(72) Erfinder: Prestel, Stephan, 76287 Rheinstetten-Mörsch (DE)
(74) Vertreter: Hemmer, Arnd

(56) Entgegenhaltungen:
- WO-A-99/49794
- US-A- 5 312 434
- US-A- 5 681 348
- US-A- 5 993 467

## Beschreibung

Die Erfindung betrifft eine medizinische Zange.

Es sind medizinische Zangen bekannt, welche einen hohlen Schaft aufweisen, in den beispielsweise ein optisches System eingesetzt werden kann. Aus der DE 44 44 403 A1 ist zum Beispiel eine medizinische Zange bekannt, welche einen hohlen Schaft aufweist, an dessen Ende ein schwenkbares Zangenmaulteil angeordnet ist. Das Öffnen und Schließen des Zangenmaulteils erfolgt über einen im Inneren des Schaftes längsbeweglichen Innenschaft. Dieser Innenschaft greift an der Oberseite des Zangenmaulteils zentral an. Das Zangenmaulteil ist im unteren Bereich an zwei Seiten drehbar an dem Außenschaft angelenkt. Durch Verschieben des Innenschaftes in Längsrichtung des Zangenschaftes wird der Angriffspunkt des Innenschaftes an dem Zangenmaulteil radial um die Drehpunkte des Zangenmaulteils verschwenkt und auf diese Weise das Zangenmaul geöffnet oder geschlossen. Im geöffneten Zustand bedingt dies, dass ein hinteres Ende des Zangenmaulteils in das Innere des Schaftes hineinverschwenkt. Dadurch wird für eine im Inneren des Innenschaftes angeordnete Optik das Sichtfeld bei geöffneter Zange sehr stark eingeschränkt.

Aus US 2001/0037128 A1 ist ein entsprechender Stand der Technik bekannt. Bei diesem ist ein rohrförmiger Innenschaft zur Bewegung der Maulteile vorgesehen, welcher über Betätigungshebel mit den Maulteilen verbunden sind. Die Betätigungshebel greifen an den Maulteilen an einer Umfangsposition zwischen den drehbaren Anlenkungspunkten der Maulteile am Zangenschaft an. Dies führt zu den oben genannten Nach-Nachteilen, dass beim Öffnen des Zangenmauls die proximalseitigen Enden der Maulteile in das Innere des Schaftes verschwenken und dort das freie Lumen einschränken.

Es ist Aufgabe der Erfindung, eine medizinische Zange zu schaffen, mit welcher eine große Zangenkraft aufgebracht werden kann und welche ein möglichst großes Sichtfeld für eine im Inneren des Zangenschaftes angeordnete Optik ermöglicht.

Diese Aufgabe wird durch eine medizinische Zange mit den in Anspruch 1 angegebenen Merkmalen gelöst. Bevorzugte Ausführungsform ergeben sich aus den Unteransprüchen.

Die erfindungsgemäße medizinische Zange weist einen rohrförmigen Außenschaft auf, an dessen distalen Ende ein Zangenmaul mit zwei Maulteilen ausgebildet ist. Der rohrförmige Außenschaft hat vorzugsweise einen kreisförmigen Querschnitt und die beiden Maulteile bilden gemeinsam vorzugsweise ebenfalls einen kreisförmigen Querschnitt von im Wesentlichen gleichem Durchmesser wie der Außenschaft. Die beiden Maulteile sind jeweils an zwei einander entgegengesetzten Seiten, d. h. vorzugsweise zwei einander diametral entgegengesetzten Seiten, drehbar an dem Außenschaft gelagert. Ferner ist in dem rohrförmigen Außenschaft ein rohrförmiger Innenschaft zur Betätigung der Maulteile angeordnet. Der rohrförmige Innenschaft erstreckt sich in Längsrichtung parallel zu dem Außenschaft. Dabei weist der rohrförmige Innenschaft vorzugsweise eine Außenkontur auf, welche korrespondierend zu der Innenkontur des Außenschaftes ausgebildet ist, so dass der Innenschaft gleitend im Inneren des Außenschaftes geführt wird. Der Innenschaft ist über zwei Hebelsysteme mit den beiden Maulteilen zu deren Betätigung gekoppelt. Die Hebelsysteme setzen die Bewegung des Innenschaftes in Längsrichtung in eine Schwenk- bzw. Drehbewegung der beiden Maulteile um. Dazu ist jeweils eines der Hebelsysteme an einer der beiden einander entgegengesetzten Seiten an den Maulteilen angelenkt. Das bedeutet, die Hebelsysteme zum Öffnen und Schließen der Maulteile sind an denselben Seiten der Zange angeordnet, an denen auch die Lagerungen für die schwenkbaren Maulteile vorgesehen sind. Die Lager- und Betätigungselemente fürdie Maulteile sind somit äußerst kompakt und dicht beieinander angeordnet. Dies ermöglicht die Lager- und Betätigungselemente für die Maulteile sehr schlank und kompakt auszugestalten. Da die Hebelsysteme seitlich an der Zange angeordnet sind, ist es nicht erforderlich, dass Elemente der Maulteile oder der Hebelsysteme beim Öffnen der Zangenmaulteile in das Innere des Außen- bzw. Innenschaftes hineinragen. Das bedeutet, der Innenraum des Innenschaftes sowie der der Öffnung des Innenschaftes an dessen distalen Ende vorgelagerte Bereich können auch bei geöffnetem Zangenmaul freigehalten werden, um ein größtmögliches Sichtfeld für eine in den Innenschaft eingesetzte Optik zu gewährleisten. Durch die beidseitige Anlenkung bzw. den beidseitigen Angriff der Hebelsysteme an jedem Maulteil kann ferner eine große Kraft auf die Maulteile übertragen werden, so dass eine große Zangenkraft beispielsweise zum Abtrennen von Gewebeteilen zur Verfügung gestellt werden kann. Die Anordnung von zwei Maulteilen ermöglicht einen sehr großen Öffnungswinkel des Zangenmauls, wodurch zum einen das Greifen schwer zugänglicher oder großer Gewebeteile begünstigt wird und zum anderen das Sichtfeld durch das geöffnete Zangenmaul hindurch sehr groß ist.

Die beiden Maulteile weisen jeweils an jeder der beiden einander entgegengesetzten Seiten sich in Längsrichtung des Außen- und Innenschaftes proximalwärts erstreckende Anlenkhebel auf, welche jeweils über eines der beiden Hebelsysteme mit dem Innenschaft gekoppelt sind. Ausgehend von einem Lagerpunkt, an dem die Maulteile an dem Außenschaft gelagert sind, erstreckt sich das Maulteil in distaler Richtung der Zange. In entgegegesetzter Richtung, d. h. in proximaler Richtung erstreckt sich ausgehend von dem Lagerpunkt der Anlenkhebel. Die Anlenkhebel sind vorzugsweise einstückig mit den zugehörigen Maulteilen ausgebildet. Zum Öffnen der Maulteile müssen somit die zugehörigen Anlenkhebel in derselben Drehrichtung wie die Maulteile verschwenkt werden. Dazu sind die Anlenkhebel über die Hebelsysteme mit dem Innenschaft verbunden. Die Hebelsysteme setzen die lineare Bewegung des Innenschaftes in seiner Längsrichtung in eine Schwenkbewegung des Anlenkhebels um, welche ihrerseits die beiden Maulteileaufschwenken. Die beiden Maulteile mit den zugehörigen Anlenkhebeln weisen somit einen Bewegungsablauf ähnlich dem einer Schere auf.

Die Anlenkhebel und das Hebelsystem sind radial weiter außenliegend als der Innenumfang des Innenschaftes angeordnet. Eine solche Anordnung bewirkt, dass der kleinste zur Verfügung stehende Innendurchmesser der gesamten Zange durch den Innendurchmesser des Innenschaftes definiert wird. Somit kann eine Optik durch das Innere des Innenschaftes bis zum distalen Ende der Zange eingeführt werden und somit sehr nahe an die distalen Enden des Zangenmauls gebracht werden. Ferner wird das Sichtfeld der Optik nicht durch weiter innenliegende Bauteile der Betätigungsmechanik für die Maulteile beeinträchtigt. Es kann somit ein sehr großes Sichtfeld geschaffen werden, welches gute Beobachtungsmöglichkeiten beim Einsatz der Zange gewährleistet.

Die beiden Maulteile sind vorzugsweise an den beiden einander entgegengesetzten Seiten jeweils an einem gemeinsamen Drehpunkt an dem Außenschaft gelagert. Diese Anordnung verringert die Anzahl der erforderlichen Drehpunkte und bewirkt günstige Hebelverhältnisse, welche eine große Kraftübertragung ermöglichen. Ferner ermöglicht diese Anordnung, dass das Zangenmaul vollständig geschlossen werden kann, so dass die beiden Maulteile mit den einander zugewandten Seitenkanten vollständig aneinander anliegen.

Die Anlenkhebel sind zweckmäßigerweise jeweils über einen Übertragungshebel mit dem Innenschaft verbunden. Diese Übertragungshebel erzeugen bei Linearbewegung des Innenschaftes an den Anlenkhebeln ein Drehmoment um deren Drehpunkt, welches zum Verschwenken der Anlenkhebel und somit der damit verbundenen Maulteile zum Öffnen und Schließen des Zangenmauls führt.

Vorzugsweise sind die Übertragungshebel jeweils an einem ersten Ende drehbar mit einem Anlenkhebel und an einem zweiten Ende drehbar mit dem Innenschaft verbunden. Auf diese Weise kann die Linearbewegung des Innenschaftes in eine Schwenk- bzw. Drehbewegung des Anlenkhebels und des damit verbundenen Maulteils umgewandelt werden. Bei einer linearen Bewegung des Innenschaftes zieht oder drückt der jeweilige Übertragungshebel den zugehörigen Anlenkhebel in einer Richtung tangential zu dessen Drehpunkt, so dass dieser um den Drehpunkt verschwenkt. Beim Verschwenken des Anlenkhebels verlagert sich dessen Kraftangriffspunkt, an dem der Übertragungshebel angreift. Diese Verlagerung kann aufgrund der drehbaren Anlenkung des Übertragungshebels an dem Anlenkhebel und dem Innenschaft ausgeglichen werden.

Weiter bevorzugt sind an den einander entgegengesetzten Seiten des Innenschaftes jeweils zwei Übertragungshebel in einem gemeinsamen Dreh- bzw. Anlenkpunkt mit dem Innenschaft verbunden. Diese Anordnung reduziert die Anzahl der erforderlichen Dreh- bzw. Gelenkverbindungen, wodurch sich die Montage vereinfacht. Ferner wird eine symmetrische Betätigung bzw. Bewegung der Übertragungshebel und somit auch der Maulteile gewährleistet. So kann eine gleichmäßige und zuverlässige Kraftübertragung sichergestellt werden. Je nach Bewegungsrichtung des Innenschaftes drücken oderziehen die Übertragungshebel an den Anlenkhebeln, wodurch die Maulteile verschwenkt werden. Dabei verläuft die Druck- bzw. Zugrichtung der Übertragungshebel unter einem Winkel zur Längsachse des Innenschaftes.

Am distalen Ende des Innenschaftes ist vorzugsweise zumindest ein radial nach außen vorstehender Vorsprung ausgebildet, welcher in eine korrespondierende Ausnehmung am distalen Ende des Außenschaftes eingreift, wobei die Ausnehmung in Längsrichtung des Außen- und des Innenschaftes eine größere Ausdehnung aufweist als der Vorsprung. Diese Anordnung von Vorsprung und Ausnehmung bildet eine Linearführung des Innenschaftes in dem Außenschaft in dessen Längsrichtung. Ferner kann aufgrund des durch die Längendifferenz von Ausnehmung und Vorsprung in Längsrichtung des Außen- und des Innenschaftes definierte Spiel eine Begrenzung für die maximale Linearbewegung des Innenschaftes in dem Außenschaft definiert werden. Eine solche Begrenzung führt aufgrund der Kopplung von Innenschaft und Maulteilen somit ebenfalls zu einer Begrenzung des Öffnungswinkels der Maulteile. Zusätzlich gewährleistet diese lineare Führung des Innenschaftes in dem Außenschaft, dass sich der Innenschaft bei einer Drehung des Außenschaftes um dessen Längsachse entsprechend mitdreht. Auf diese Weise ist es möglich, Außen- und Innenschaft drehbar an einem Zangengriff anzuordnen, wobei der gesamte Maulbereich der Zange, d. h. das distale Ende der Zange mit dem Zangenmaul sehr einfach durch Drehung des Außenschaftes um dessen Längsachse verdreht werden kann.

Weiter bevorzugt ist an dem Innenschaft jeweils ein Vorsprung an jeder der beiden einander entgegengesetzten Seiten ausgebildet und der Außenschaft weist zwei korrespondierende Ausnehmungen an den einander entgegengesetzten Seiten auf, in welche die Vorsprünge eingreifen. Auf diese Weise wird eine präzise Führung des Innenschaftes in dem Außenschaft erreicht. Ferner kann eine sichere Kraftübertragung bei Drehung des Außenschaftes auf den Innenschaft gewährleistet werden. Durch die Anordnung von Ausnehmung und Vorsprung an den beiden einander entgegengesetzten Seiten, an denen auch die Lagerpunkte sowie die Hebelsysteme zum Verschwenken der Maulteile vorgesehen sind, wird eine äußerst kompakte und schlanke Anordnung sämtlicher Führung- bzw. Lager- und Betätigungselemente erreicht. Auf diese Weise kann ein größtmöglicher Freiraum im Inneren der Zange in deren distalen Endbereich geschaffen werden. Ferner ist es möglich, Öffnungen bzw. Ausnehmungen in Außen- und Innenschaft in deren distalen Endbereiche vorzusehen.

Durch diese Maßnahmen kann das Sichtfeld für eine im Inneren der Zange angeordnete Optik weiter vergrößert werden.

Vorzugsweise sind die Anlenkhebel der beiden Maulteile auf jeder der beiden einander entgegengesetzten Seiten derart ausgebildet, dass der Anlenkhebel des ersten Maulteils radial weiter innenliegend ausgebildet ist als der Anlenkhebel des zweiten Maulteils. Das bedeutet, die beiden Anlenkhebel sind in radialer Richtung, d. h. in der Richtung, welche die beiden entgegengesetzten Seiten quer zur Längsrichtung des Außen- und Innenschaftes miteinander verbindet, übereinanderliegend bzw. überlappend angeordnet. Diese scherenartige Anordnung der Hebel ermöglicht einen äußerst schmalen und kompakten Aufbau des Hebelsystems zur Betätigung der Maulteile.

Weiter bevorzugt sind die zwei Übertragungshebel an jeder der beiden einander entgegengesetzten Seiten jeweils radial übereinanderliegend angeordnet. Die Anordnung ist vorzugsweise so ausgebildet, dass derjenige Übertragungshebel, welcher mit dem Anlenkhebel des ersten Maulteils verbunden ist, in einer Ebene mit dem Anlenkhebel des zweiten Maulteils liegt, während derjenige Übertragungshebel, welcher mit dem Anlenkhebel des zweiten Maulteils verbunden ist, in einer Ebene mit dem Anlenkhebel des ersten Maulteils liegt. Anlenk- und Übertragungshebel sind somit versetzt zueinander übereinanderliegend bzw. überlappend angeordnet. Es wird somit ein parallelogrammartiges Hebelsystem durch die Anlenk- und Übertragungshebel für die beiden Maulteile an jeder der beiden einander entgegengesetzten Seiten geschaffen. Diese Anordnung ist äußerst kompakt und kann ferner eine große Kraft von dem Innenschaft auf die Maulteile übertragen, so dass diese eine große Schließkraft ausüben können, was ein Abtrennen von Gewebeteilen begünstigt.

Ferner sind die Anlenkhebel der beiden Maulteile vorzugsweise an jeder derzwei einander entgegengesetzten Seiten derart ausgebildet, dass sich in der Längsrichtung des Außenschaftes gesehen im geschlossenen Zustand des Zangenmauls das freie Ende des Anlenkshebels des ersten Maulteils in Verlängerung des zweiten Maulteils und das freie Ende des Anlenkhebels des zweiten Maulteils in Verlängerung des ersten Maulteils erstreckt. Das bedeutet, die Anlenkhebel sind bezüglich der Längsachse des Außen- und Innenschaftes abgewinkelt bzw. gestuft zur Längsachse des jeweiligen Maulteils angeordnet. Vorzugsweise erstreckt sich der Anlenkhebel parallel versetzt zur Längsrichtung des zugehörigen Maulteils, wobei der Drehpunkt des Maulteils vorzugsweise im Wesentlichen auf der Mitte zwischen der Längsachse des Anlenkhebels und der Längsachse des Maulteils liegt. Die Längsachse des Anlenkhebels und die Längsachse des Maulteils verlaufen dabei im Wesentlichen parallel zur Längsachse des Außen- und Innenschaftes. Die Längsachse des Innenschaftes liegt vorzugsweise im Wesentlichen in einer Ebene mit den beiden Drehpunkten, an denen die beiden Maulteile an dem Außenschaft angelenkt sind. Auch die beiden Drehpunkte, an denen die Übertragungshebel an den Innenschaft schwenkbar gelagert sind, liegen vorzugsweise in der Ebene der Längsachse des Innenschaftes und der Drehpunkte der Maulteile. Die Endbereiche der Anlenkhebel, welche mit den Übertragungshebeln verbunden sind, liegen vorzugsweise außerhalb dieser Ebene. Auf diese Weise wird sichergestellt, dass die Längsrichtung der Übertragungshebel, d. h. die Richtung der von dem Übertragungshebel übertragenen Kraft, immer unter einem Winkel zur Längsachse der zugehörigen Anlenkhebel und des Innenschaftes verläuft. Auf diese Weise kann sichergestellt werden, dass in jeder Stellung der Zange ein sicheres Verschwenken der Maulteile möglich ist und sich die Zange nicht verklemmen kann.

In einer bevorzugten Ausführungsform kann in zumindest einem der beiden Maulteile ein Loch ausgebildet sein. Ein solches Loch ermöglicht es, vom Inneren der Zange bzw. des Innenschaftes auch bei geschlossenem Zangenmaul durch dieses hindurchzusehen. Auf diese Weise kann auch bei geschlossenem Zangenmaul die Positionierung der Zange bei einer Operation erleichtert werden. Das Loch ist vorzugsweise in der Mitte des Maulteils beabstandet von dessen Außen- bzw. Schneidkante angeordnet, so dass weiterhin ein sicherer Griff bzw. ein zuverlässiges Schneiden bzw. Abtrennen von Gewebe durch die Zange gewährleistet wird.

Nachfolgend wird die Erfindung beispielhaft anhand der beigefügten Figuren beschrieben. In diesen zeigt:
- Fig. 1: eine Schnittansicht der erfindungsgemäßen medizinischen Zange,
- Fig. 2: eine Detailansicht des Zangenmauls im geschlossenen Zustand,
- Fig. 3: eine Detailansicht des Zangenmauls im geöffneten Zustand,
- Fig. 4: eine vergrößerte schematische Schnittansicht des Zangenmauls im geschlossenen Zustand,
- Fig. 5: eine Ansicht des Zangenmauls in Richtung der Pfeile A in Fig. 4,
- Fig. 6: eine Ansicht des Zangenmauls gemäß Fig. 4 im geöffneten Zustand,
- Fig. 7: eine Detailansicht eines Maulteils in Draufsicht,
- Fig. 8: eine Schnittansicht eines Maulteils entlang der Linie I-I in Fig. 7, und
- Fig. 9: eine Detailansicht eines Umlenkhebels.

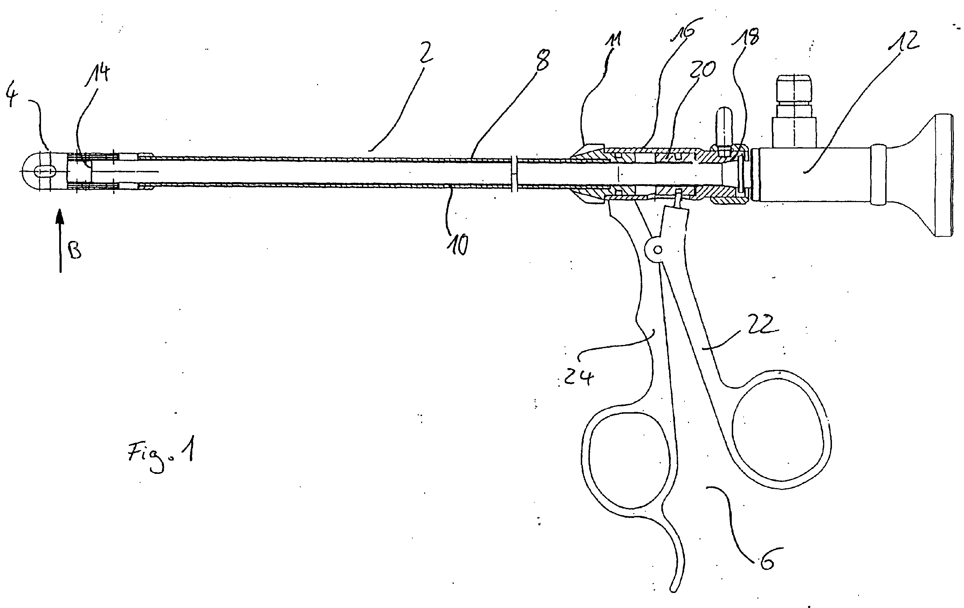

Fig. 1 zeigt in einer Schnittansicht eine Gesamtansicht der erfindungsgemäßen Zange. Die medizinische Zange weist einen Zangenschaft 2 auf, an dessen distalen Ende ein Zangenmaul 4 und an dessen proximalen Ende eine Zangenhandhabe 6 angeordnet ist. Der Zangenschaft 2 besteht aus einem Außenschaft 8 und einem im Inneren des Außenschaftes angeordneten Innenschaft 10. Der Außenschaft 8 und der Innenschaft 10 sind rohrförmig, insbesondere mit einem kreisförmigen Querschnitt ausgebildet. Dabei weist der Innenschaft 10 einen Außendurchmesser auf, welcher im Wesentlichen dem Innendurchmesser des Außenschaftes 8 entspricht. Dies ermöglicht, dass der Innenschaft 10 in dem Außenschaft 8 passend im Wesentlichen spielfrei oder mit geringem Spiel geführt wird. Im Inneren des hohlen Innenschaftes 10 ist eine Optik 12 angeordnet. Die Optik 12 erstreckt sich vom proximalen Ende der Zange in den Innenschaft 10 hinein und durch diesen hindurch vorzugsweise bis zum distalen Ende 14 des Innenschaftes 10, um eine Beobachtung des das Zangenmaul 4 umgebenden Bereiches bzw. des dem Zangenmaul 4 vorgelagerten Bereichs bei einer Operation zu ermöglichen.

Am proximalen Ende der Zange ist ein Zangengehäuse 16 vorgesehen, von dem ausgehend sich der Zangenschaft 2 in distaler Richtung erstreckt. In dem Zangengehäuses 16 ist am distalen Ende ein drehbares Handrad 11 angeordnet, welches fest mit dem Außenschaft 8 verbunden ist. Durch Drehen des Handrades 11 kann der Außenschaft 8 um seine Längsachse gedreht werden. Am proximalen Ende des Zangengehäuses 16 ist eine Kegelaufnahme 18 ausgebildet, in der die Optik 12 lösbar fixiert werden kann. Ferner ist im Inneren des Zangengehäuses 16 eine Schiebehülse 20 angeordnet. Die Schiebehülse 20 ist in dem Zangengehäuse 16 in Längsrichtung des Außenschaftes 8 und des Innenschaftes 10 verschiebbar. Die Schiebehülse 20 ist mit dem beweglichen Handhabenteil 22 zur Betätigung der Schiebehülse 20 verbunden. Der bewegliche Handhabenteil 22 ist schwenkbar an dem feststehenden Handhabenteil 24 gelagert. Der feste Handhabenteil 24 ist mit dem Zangengehäuse 16 verbunden. Durch Verschwenken des beweglichen Handhabenteils 22 wird die Schiebehülse 20 in axialer Richtung, d. h. in Längsrichtung des Zangenschaftes 2 in dem Zangengehäuse 16 verschoben. Die Schiebehülse 20 ist fest mit dem Innenschaft 10 verbunden. So wird bei Betätigung des beweglichen Handhabenteils 22 der Innenschaft 10 in seiner Längsrichtung, d.h. in axialer Richtung des Zangenschaftes 2 in dem Außenschaft 18 verschoben.

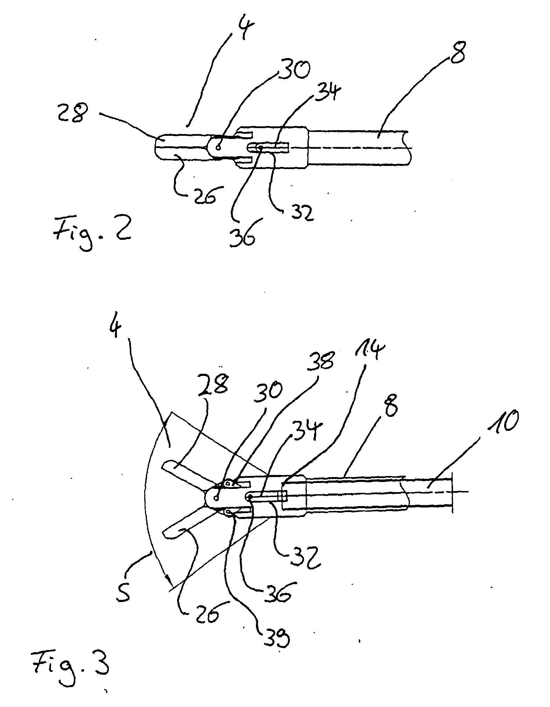

Fig. 2 zeigt eine Detailansicht des Zangenmauls 4 im geschlossenen Zustand. Fig. 2 ist eine Seitenansicht des Zangenmauls 4 in Richtung des Pfeils B in Fig. 1. Das Zangenmaul 4 wird durch zwei Maulteile 26, 28 gebildet. Die beiden Maulteile 26 und 28 sind schaufel- bzw. schalenförmig ausgebildet, so dass sie gemeinsam im geschlossenen Zustand ebenfalls einen im Wesentlichen rohrförmigen Querschnitt definieren. Das bedeutet, die Maulteile 26 und 28 schließen im geschlossenen Zustand einen Freiraum ein. Die Maulteile 26 und 28 sind an zwei diametral entgegengesetzten Seiten jeweils an einem Drehpunkt 30 an dem Außenschaft 8 drehbar gelagert. Dabei sind die Maulteile 26 und 28 an jeder Seite jeweils in einem gemeinsamen Drehpunkt 30 gelagert. Ferner ist im Bereich seines distalen Endes an dem Außenschaft 8 eine Längsnut 32 ausgebildet, welche sich in Längsrichtung des Außenschaftes 8 erstreckt. An jeder der diametral entgegengesetzten Seiten, an der auch die Drehpunkte 30 vorgesehen sind, ist jeweils eine Längsnut 32 ausgebildet. In diese Längsnuten 32 greifen längliche Vorsprünge 34 ein. Die Vorsprünge 34 erstrecken sich ebenfalls in Längsrichtung des Zangenschaftes 2 bzw. des Außenschaftes 8. Die Vorsprünge 34 sind fest im Bereich des distalen Endes 14 des Innenschaftes 10 an diesem ausgebildet und erstrecken sich von diesem radial nach außen. Die Längsnuten 32 sind in ihrer Längsrichtung länger ausgebildet als die Vorsprünge 34, so dass die Vorsprünge 34 in Längsrichtung in den Längsnuten 32 beweglich sind. Auf diese Weise werden die Vorsprünge 34 und somit der Innenschaft 10 bei einer Bewegung in Längsrichtung des Zangenschaftes 2 in den Längsnuten 32 geführt. Ferner bewirken die Längsnuten 32, dass bei einer Drehung des Außenschaftes 8 über das Handrad 11 der Innenschaft 10 entsprechend mitgedreht wird, so dass der gesamte Zangenschaft 2 mit dem Zangenmaul 4 bezüglich des Zangengehäuses 16 um die Längsachse des Zangenschaftes 2 verdreht werden kann.

Fig. 3 zeigt eine Ansicht des Zangenmauls 4 entsprechend Fig. 2, wobei das Zangenmaul 4 geöffnet ist. Zum Öffnen des Zangenmauls 4 wird der Innenschaft 10 in seiner Längsrichtung im Inneren des Außenschaftes 8 in distaler Richtung verschoben. Dabei verschieben sich auch die Vorsprünge 34 in den Längsnuten 32. Bei entsprechender Dimensionierung der Länge der Längsnuten 32 und der korrespondierenden Vorsprünge 34 können die Stirnflächen der Längsnuten 32 und der Vorsprünge 34 als Anschläge zur Begrenzung der Bewegung des Innenschaftes 10 in dessen Längsrichtung wirken. An den Vorsprüngen 34 ist jeweils ein Anlenkpunkt 36 ausgebildet. Insgesamt sind somit zwei Anlenkpunkte 36 an den zwei diametral entgegengesetzten Seiten des Zangenschaftes 2 vorgesehen, an denen auch die Drehpunkte 30 angeordnet sind. Die Anlenkpunkte 36 liegen jeweils mit einem der Drehpunkte 30 auf einer gemeinsamen Achse, welche sich parallel zur Längsachse des Zangenschaftes 2 an dessen Seite erstreckt. An den Anlenkpunkten 36 sind Übertragungshebel 38 und 39 angelenkt, welche die Maulteile 26 und 28 um deren Drehpunkte 30 verschwenken. Ferner ist in Fig. 3 das maximale Sichtfeld S für eine im Inneren des Innenschaftes 10 angeordnete Optik gekennzeichnet. Dieses Sichtfeld S erstreckt sich ausgehend vom distalen Ende 14 des Innenschaftes 10. Ein derart großes Sichtfeld S wird dadurch ermöglicht, dass sämtliche Lager-und Betätigungselemente, d. h. die Übertragungshebel 38, 39 die Drehpunkte 30 sowie die Anlenkpunkte 36 an zwei einander diametral entgegengesetzten Seiten des Außenschaftes 8 angeordnet sind. Dies ermöglicht, dass zwischen diesen beiden entgegengesetzten Seiten bzw. Seitenbereichen in dem Außenschaft 8 Öffnungen ausgebildet werden, welche ein entsprechend großes Sichtfeld S ermöglichen.

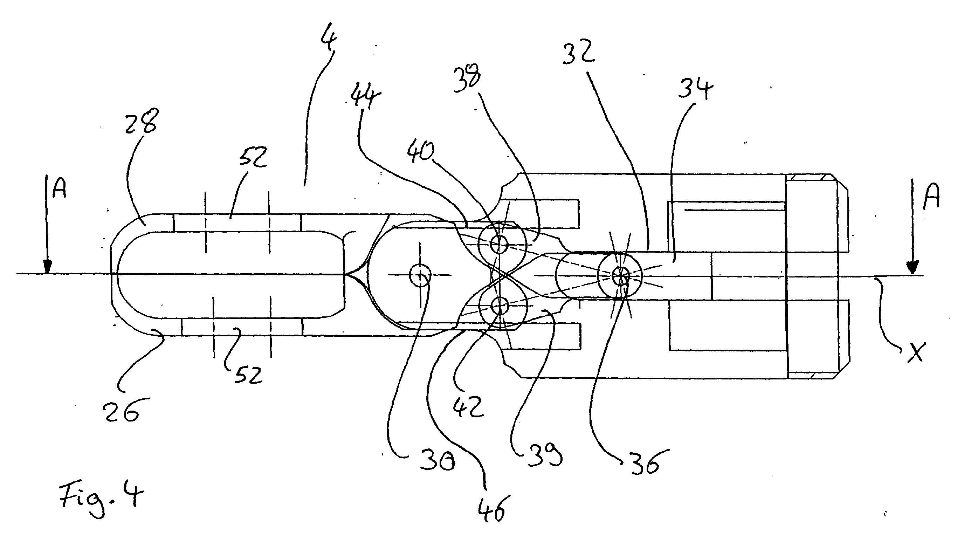

Der genaue Aufbau des Hebelsystems zur Betätigung der Maulteile 26 und 28 wird anhand der schematischen Darstellung in Fig. 4 näher erläutert. Die Ansicht in Fig. 4 entspricht im Wesentlichen der Ansicht gemäß Fig. 2, das Zangenmaul 4 ist jedoch geschnitten dargestellt, um die einzelnen Hebelelemente sichtbar zu machen. Die Maulteile 26 und 28 sind im geschlossenen Zustand dargestellt. Da die Maulteile 26, 28 jeweils an zwei diametral entgegengesetzten Seiten des Zangenschaftes 2 angelenkt und gelagert sind, sind sämtliche Hebel- und Betätigungselemente in identischer Weise an den beiden diametral entgegengesetzten Seiten des Zangenschaftes 2 ausgebildet. Aus diesem Grunde wird nachfolgend nur die Ausgestaltung an einer der beiden Seiten näher erläutert, die Hebelelemente an der entgegengesetzten Seite sind identisch ausgebildet. Der in Längsrichtung des Zangenschaftes 2 in der Längsnut 32 axial bewegliche Vorsprung 34 ist fest mit dem Innenschaft 10 (in Fig. 4 nicht dargestellt) verbunden. An dem Vorsprung 34 ist ein Anlenkpunkt 36 ausgebildet. An diesem Anlenkpunkt 36 sind die beiden Übertragungshebel 38 und 39 schwenkbar angelenkt bzw. gelagert. Dazu ist in dem Vorsprung 34 und in den Übertragungshebeln 38, 39 jeweils eine Bohrung vorgesehen, durch die sich ein Niet bzw. Lagerbolzen erstreckt. Die Übertragungshebel 38 und 39 sind gewinkelt ausgebildet, so dass sie im eingebauten Zustand in zwei entgegengesetzte Richtungen zu der Längsachse x bzw. einer Ebene durch die Längsachse x und die Drehpunkte 30 abgewinkelt sind. An dem den Anlenkpunkten 36 entgegengesetzten Enden der Übertragungshebel 38 und 39 sind Drehpunkte 40 bzw. 42 vorgesehen. Aufgrund der gewinkelten Ausgestaltung der Übertragungshebel 38 und 39 liegen die Drehpunkte 40 und 42 außerhalb der Ebene bzw. Achse, welche sich durch die Drehpunkte 30 und Anlenkpunkte 36 erstreckt.

In den Drehpunkten 40 und 42 sind die Übertragungshebel 38 und 39 drehbar mit den Maulteilen 26 und 28 verbunden. Dies kann ebenfalls durch Lagerbolzen oder Niete erreicht werden, die in entsprechende Löcher in den Übertragungshebeln 38,39 und den Maulteilen 26, 28 eingesetzt sind. Die Maulteile 26 und 28 weisen Anlenkhebel 44 und 46 auf. Der Anlenkhebel 44 ist einstückig mit dem Maulteil 26 und der Anlenkhebel 46 einstückig mit dem Maulteil 28 ausgebildet. Die Anlenkhebel 42 und 44 erstrecken sich ausgehend von den zugehörigen Maulteilen 26 und 28 in entgegengesetzter, d. h. in proximaler Richtung der Zange über den Drehpunkt 30 hinaus. Dabei sind die Anlenkhebel 44 und 46 gegenüber den zugehörigen Maulteilen 26 und 28 derart abgewinkelt, dass das Maulteil 26 über den Anlenkhebel 44 an dem Drehpunkt 40 an der dem Maulteil 26 entgegengesetzten bzw. gegenüberliegenden Seite bezüglich der Längsachse x angelenkt ist. Entsprechend ist das Maulteil 28 über den Anlenkhebel 46 an dem Drehpunkt 42 an der dem Maulteil 28 bezüglich der Längsachse x gegenüberliegenden Seite angelenkt. Auf diese Weise wird eine Parallelogramm-Mechanik geschaffen, wobei die Drehpunkte 40 und 42 in einer Richtung normal zu der Längsachse x voneinander und von dieser beabstandet sind. Dies bewirkt, dass die Verbindungslinien zwischen den Drehpunkten 40 und 42 mit dem Drehpunkt 30 sowie dem Anlenkpunkt 36 in einem Winkel zur Längsachse x verlaufen. Dies stellt sicher, dass bei einer Bewegung des Anlenkpunktes 36 in Richtung der Längsachse x in distaler Richtung die beiden Drehpunkte 40 und 42 durch die Übertragungshebel 36 und 39 auseinandergedrückt werden, so dass sie sich voneinander entfernen. Bei dieser Bewegung der Drehpunkte 40 und 42 werden die mit diesen verbundenen Anlenkhebel 44 und 46 und somit ebenfalls die zugehörigen Maulteile 26 und 28 um den Drehpunkt 30 verschwenkt, so dass sich das Zangenmaul 4 öffnet. Die Bewegung des Anlenkpunktes 36 in Längsrichtung x erfolgt durch eine Bewegung des Innenschaftes 10 in seiner Längsrichtung, da der Innenschaft 10 fest mit dem Vorsprung 34 verbunden ist.

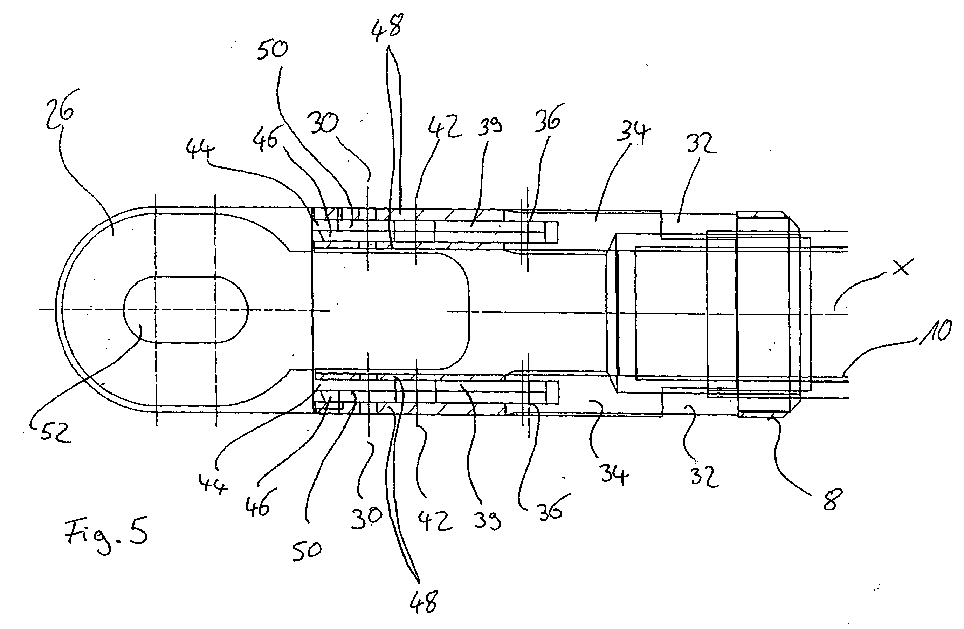

Fig. 5 zeigt eine Schnittansicht des Zangenmauls gemäß Fig. 4 in Richtung der Pfeile A in Fig. 4. Der distale Endbereich 48 des Außenschaftes 8 bildet an zwei einander diametral entgegengesetzten Seiten jeweils einen Aufnahmespalt 50, in welchem die Anlenk- und Übertragungshebel 44, 46 bzw. 38 und 39 angeordnet sind. Die Hebel sind derart übereinanderliegend bzw. überlappend angeordnet, dass der Übertragungshebel 39 an einer der beiden diametral entgegengesetzten Seiten radial weiter innen liegend bezüglich der Längsachse x angeordnet ist als an der entgegengesetzten Seite. Entsprechend sind die zugehörigen Anlenkhebel 46 an einer Seite radial weiter innenliegend angeordnet als an der anderen Seite. So bilden die Anlenk- und Übertragungshebel 38, 39, 44 und 46 eine äußerst flache Parallelogramm-Mechanik, welche in dem jeweiligen Aufnahmespalt 50 angeordnet ist. Zu deren Ausgestaltung ist auch jeweils einer der Anlenkhebel 44 bzw. 46 an den Maulteilen 26 und 28 an der einen Seite weiter innenliegend zu der Längsachse x gelegen als an der diametral entgegengesetzten Seite. An der Seite, an welcher der Anlenkhebel 46 weiter außen liegt, liegt der zugehörige Übertragungshebel 39 weiter innen und an der Seite, an welcher der Anlenkhebel 46 weiter innen liegt, liegt der zugehörige Übertragungshebel 39 weiter außen. Entsprechendes gilt für die Anlenkhebel 44 des Maulteils 26 und die zugehörigen Übertragungshebel 38. In den Aufnahmespalten 50 sind die Anlenkhebel 44, 46 und Übertragungshebel 38, 39 jeweils parallel zueinander übereinanderliegend bzw. überlappend angeordnet.

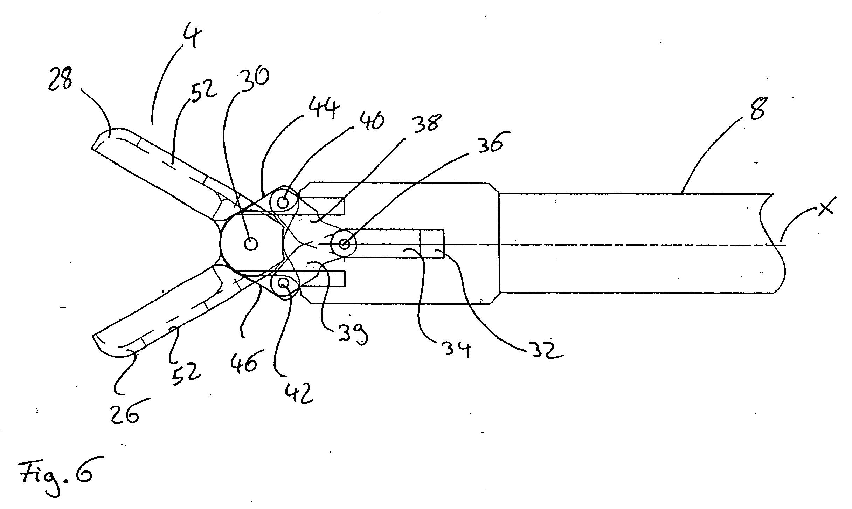

Fig. 6 zeigt eine Ansicht entsprechend Fig. 4, bei welcher das Zangenmaul 4 geöffnet ist. Wie bereits anhand von Fig. 4 erläutert, wird zum Öffnen des Zangenmauls 4 der Innenschaft 10 (in Fig. 6 nicht gezeigt) im Inneren des Außenschaftes 8 entlang der Längsachse x in Richtung des distalen Endes der Zange, d. h. in Richtung des Zangenmauls 4 bewegt. Dabei bewegt sich an jeder der diametral entgegengesetzten Seiten der Vorsprung 34 mit dem Anlenkpunkt 36 ebenfalls in Richtung des distalen Endes. Dadurch werden an jeder Seite die Übertragungshebel 38 und 39 nach außen gedrückt, so dass sich die Drehpunkte 40 und 42 voneinander entfernen. Da die Anlenkhebel 44 und 46 an den Drehpunkten 40 und 42 mit den Übertragungshebeln 38 und 39 drehbar verbunden sind, werden bei dieser Bewegung die Anlenkhebel 44 und 46 um den jeweiligen Drehpunkt 30 gedreht. Dabei werden ebenfalls die Maulteile 26 und 28 um die beiden Drehpunkte 30 gedreht, so dass sich das Zangenmaul 4 öffnet.

Fig. 7 zeigt eine Einzelansicht eines Maulteils 26. Das Maulteil 28 ist identisch zu dem Maulteil 26 ausgebildet. Das Maulteil 26 ist schalenförmig, wobei am tiefsten Punkt eine Öffnung 52 ausgebildet ist. Die Öffnung 52 ermöglicht, dass bei geschlossenem Zangenmaul 4 durch das Innere der Zange der Bereich der Umgebung des Zangenmauls 4 durch die Öffnung 52 hindurch betrachtet werden kann. Am distalen Ende des Maulteils 26 erstrecken sich in proximaler Richtung die Anlenkhebel 44. Die Anlenkhebel 44 erstrecken sich parallel zu der Längsachse x. In den Anlenkhebeln 44 sind entsprechende Bohrungen für die Drehpunkte 30 und 42 zum Verbinden mit dem Außenschaft 8 und den Übertragungshebeln 39 ausgebildet. Die beiden Anlenkhebel 44 sind an zwei diametral entgegengesetzten Seiten des Maulteils 26 ausgebildet. Dabei ist an einer Seite der Anlenkhebel 44a weiter innenliegend, d. h. näher zu der Längsachse x angeordnet als der Anlenkhebel 44b an der diametral entgegengesetzten Seite. Diese Ausgestaltung ermöglicht die versetzt übereinanderliegende Anordnung von Anlenk- und Übertragungshebeln. Die Übertragungshebel 46 an dem zweiten Zangenmaul 28 sind identisch ausgestaltet.

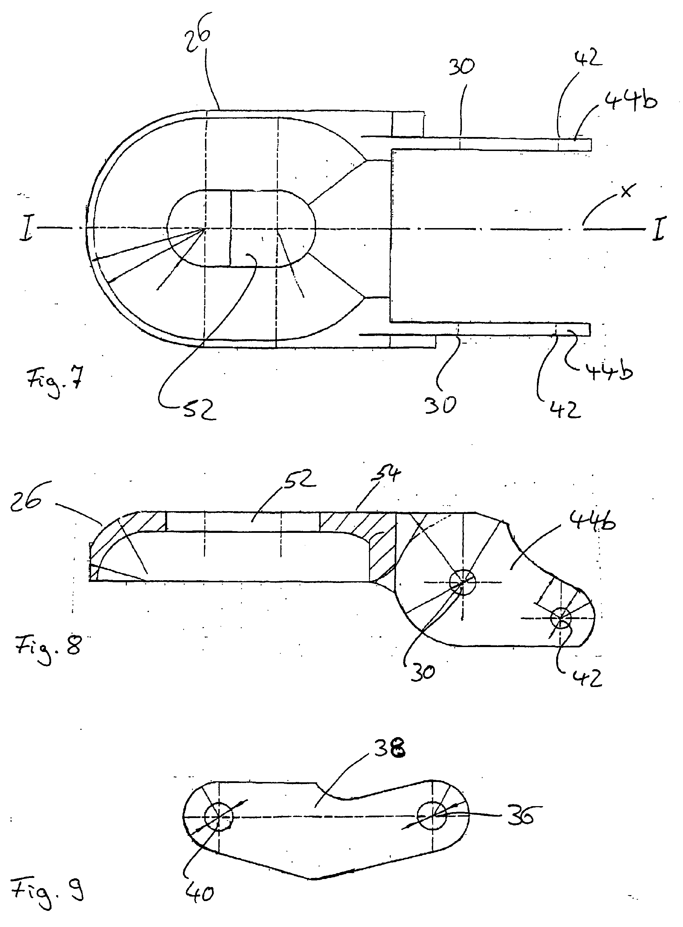

Fig, 8 zeigt eine Schnittansicht des Maulteils 26 gemäß Fig. 7 entlang Linie I-I in Fig. 7. In Fig. 8 ist die schalenförmige Ausgestaltung des Maulteils 26 zu erkennen, wobei die Öffnung 52 am tiefsten Punkt, d. h. an demjenigen Punkt, welcher am weitesten entfernt von der Mittelachse des Zangenschaftes 2 gelegen ist, angeordnet ist. Der Anlenkhebel 44 bzw. 44 b ist abgewinkelt zu dem Maulteil 26 ausgebildet, so dass der Drehpunkt 42 radial weiter von der Außenseite 54 des Maulteils 26 entfernt ist als der Drehpunkt 30. Dies ermöglicht, dass die beiden Maulteile 26 und 28 derart übereinander angeordnet werden, dass sie um denselben gemeinsamen Drehpunkt 30 drehbar sind und sich die beiden Anlenkhebel 44 und 46 überkreuzen. Auf diese Weise kann die anhand von Fig. 4 erläuterte Parallelogramm-Mechanik geschaffen werden, welche eine sichere Betätigung des Zangenmauls 4 ohne Gefahr eines Verklemmens ermöglicht.

Fig. 9 zeigt eine Detailansicht eines Übertragungshebels 39. Die Übertragungshebel 38 sind identisch ausgebildet. Der Übertragungshebel 38 weist an einem Ende eine Öffnung bzw. Bohrung für den Drehpunkt 40 und am entgegengesetzten Ende ein Loch bzw. eine Bohrung für den Anlenkpunkt 36 auf. Durch diese Löcher können sich Lager- bzw. Gelenkbolzen oder Niete erstrecken. Der Anlenkhebel 38 weist eine geknickte Ausgestaltung auf, wie anhand von Fig. 4 erläutert.

Insgesamt weist die erfindungsgemäße medizinische Zange eine aäußerst schmale Anordnung sämtlicher Lager- und Betätigungselemente für das Zangenmaul 4 an zwei diametral entgegengesetzten Seiten des Zangeschaftes 2 auf. Insbesondere sind die Betätigungselemente, d.h. die Anlenkhebel 44, 46 und die Übertragungshebel jeweils im selben Umfangsbereich wie die Drehpunkte 30, an denen die Maulteile 26, 28 gelagert sind, angeordnet. Auf diese Weise können die Umfangsbereiche, die zur Lagerung und Anlenkung der Maulteile26, 28 an dem Zangenschaft 2 erforderlich sind, sehr schlank ausgestaltet werden, so dass in den übrigen Umfangsbereichen das Sichtfeld S einer in dem Zangenschaft 2 angeordneten Optik 12 nicht beeinträchtigt wird. Ferner ermöglicht die beidseitige Anlenkung der Maulteile 26 und 28, eine große Klemmkraft der Zange zu erzeugen, wobei beide Maulteile 26, 28 sehr verwindungssteif geführt werden.

### Bezugszeichenliste

- 2 -: Zangenschaft
- 4 -: Zangenmaul
- 6 -: Handhabe
- 8 -: Außenschaft
- 10 -: Innenschaft
- 11 -: Handrad
- 12 -: Optik
- 14 -: distales Ende des Innenschaftes
- 16 -: Zangengehäuse
- 18 -: Kegelverbindung
- 20 -: Schiebehülse
- 22 -: bewegliches Handhabenteil
- 24 -: festes Handhabenteil
- 26, 28 -: Maulteile
- 30 -: Drehpunkt
- 32 -: Längsnut
- 34 -: Vorsprung
- 36 -: Anlenkpunkt
- 38, 39 -: Übertragungshebel
- 40, 42 -: Drehpunkte
- 44, 46 -: Anlenkhebel
- 48 -: distaler Endbereich des Außenschaftes
- 50 -: Aufnahmespalt
- 52 -: Öffnung
- 54 -: Außenseite

## Patentansprüche

1. Medizinische Zange mit
einem rohrförmigen Außenschaft (8), an dessen distalen Ende ein Zangenmaul (4) mit zwei Maulteilen (26, 28) ausgebildet ist, wobei die beiden Maulteile (26, 28) jeweils an zwei einander entgegengesetzten Seiten drehbar an dem Außenschaft (8) gelagert sind, einem rohrförmigen Innenschaft (10) zur Betätigung der Maulteile (26, 28), welcher im Inneren des Außenschaftes (8) in seiner Längsrichtung (x) verschiebbar angeordnet ist, wobei der Innenschaft (10) über zwei Hebelsysteme (38, 39) mit den beiden Maulteilen (26, 28) zu deren Betätigung gekoppelt ist, **dadurch gekennzeichnet, dass**
die beiden Maulteile (26,28) jeweils an jeder der beiden einander entgegengesetzten Seiten sich in Längsrichtung (x) des Außen- (8) und Innenschaftes (10) proximalwärts erstreckende Anlenkhebel (44, 46) aufweisen, welche jeweils über eines der beiden Hebelsysteme (38, 39) mit dem Innenschaft (10) gekoppelt sind, und
die Anlenkhebel (44, 46) und das Hebelsystem (38, 39) radial weiter außen liegend als der Innenumfang des Innenschaftes (10) angeordnet sind.

2. Medizinische Zange nach Anspruch 1, **dadurch gekennzeichnet, dass** die beiden Maulteile (26, 28) an den beiden einander entgegengesetzten Seiten jeweils an einem gemeinsamen Drehpunkt (30) an dem Außenschaft (8) gelagert sind.

3. Medizinische Zange nach Anspruch 1 oder 2, **dadurch gekennzeichnet, dass** die Anlenkhebel (44, 46) jeweils über einen Übertragungshebel (38, 39) mit dem Innenschaft (10) verbunden sind.

4. Medizinische Zange nach Anspruch 3, **dadurch gekennzeichnet, dass** die Übertragungshebel (38, 39) jeweils an einem ersten Ende (40, 42) drehbar mit einem Anlenkhebel (44, 46) und an einem zweiten Ende (36) drehbar mit dem Innenschaft verbunden sind.

5. Medizinische Zange nach Anspruch 4, **dadurch gekennzeichnet, dass** an den einander entgegengesetzten Seiten des Innenschaftes (10) jeweils zwei Übertragungshebel (38, 39) in einem gemeinsamen Drehpunkt (39) mit dem Innenschaft (10) verbunden sind.

6. Medizinische Zange nach einem der vorangehenden Ansprüche, **dadurch gekennzeichnet, dass** am distalen Ende (14) des Innenschaftes (10) zumindest ein radial nach außen vorstehender Vorsprung (34) ausgebildet ist, welcher in eine korrespondierende Ausnehmung (32) am distalen Ende des Außenschaftes (8) eingreift, wobei die Ausnehmung (32) in Längsrichtung des Außen-(8) und des Innenschaftes (10) eine größere Ausdehnung aufweist als der Vorsprung (34).

7. Medizinische Zange nach Anspruch 6, **dadurch gekennzeichnet, dass** an dem Innenschaft (10) jeweils ein Vorsprung (34) an jeder der beiden einander entgegengesetzten Seiten ausgebildet ist und der Außenschaft (8) zwei korrespondierende Ausnehmungen (32) an den einander entgegengesetzten Seiten aufweist, in welche die Vorsprünge (34) eingreifen.

8. Medizinische Zange nach einem der vorangehenden Ansprüche, **dadurch gekennzeichnet, dass** die Anlenkhebel (44, 46) der beiden Maulteile (26, 28) auf jeder der beiden einander entgegengesetzten Seiten derart ausgebildet sind, dass der Anlenkhebel (44) des ersten Maulteils (26) radial weiter innen liegend ausgebildet ist als der Anlenkhebel (46) des zweiten Maulteils (28).

9. Medizinische Zange nach einem der Ansprüche 3 bis 7, **dadurch gekennzeichnet, dass** die zwei Übertragungshebel (38, 39) an jeder der beiden einander entgegengesetzten Seiten jeweils radial übereinanderliegend angeordnet sind.

10. Medizinische Zange nach einem der vorangehenden Ansprüche, **dadurch gekennzeichnet, dass** die Anlenkhebel (44, 46) der beiden Maulteile (26, 28) an jeder der zwei einander entgegengesetzten Seiten derart ausgebildet sind, dass sich in der Längsrichtung des Außenschaftes (8) gesehen im geschlossenen Zustand des Zangenmauls (4) das freie Ende des Anlenkhebels (44) des ersten Maulteils (26) in Verlängerung des zweiten Maulteils (28) und das freie Ende des Anlenkhebels (46) des zweiten Maulteils in (28) Verlängerung des ersten Maulteils (26) erstreckt.

11. Medizinische Zange nach einem der vorangehenden Ansprüche, **dadurch gekennzeichnet, dass** in zumindest einem der beiden Maulteile (26, 28) ein Loch (52) ausgebildet ist.

## Claims

1. A medical forceps
with a tubular outer shank (8) on whose distal end a forceps jaw (4) with two jaw parts (26, 28) is formed, wherein the two jaw parts (26, 28) are rotatably mounted on the outer shank (8) in each case on two sides opposite one another,
with a tubular inner shank (10) for actuating the jaw parts (26, 28), which is displaceably arranged in the inside of the outer shank (8) in its longitudinal direction (x), wherein the inner shank (10) is coupled to the two jaw parts (26, 28) for their actuation via two lever systems (38, 39), **characterised in that**
the two jaw parts (26, 28) in each case on each of the two sides opposite one another comprise linkage levers (44, 46) which extend proximally in the longitudinal direction (x) of the outer-(8) and inner shank (10) and which in each case are connected to the inner shank (10) via one of the two lever systems (38, 39), and the linkage levers (44, 46) and the lever system (38, 39) are arranged lying further radially outwards than the inner periphery of the inner shank (10).

2. A medical forceps according to claim 1, **characterised in that** the two jaw parts (26, 28) on the two sides opposite one another in each case are mounted on a common rotation point (30) on the outer shank (8).

3. A medical forceps according to claim 1 or 2, **characterised in that** the linkage levers (44, 46) in each case are connected to the inner shank (10) via a transmission lever (38, 39).

4. A medical forceps according to claim 3, **characterised in that** the transmission levers (38, 39) in each case at a first end (40, 42) are rotatably connected to a linkage lever (44, 46) and at a second end (36) rotatably to the inner shank.

5. A medical forceps according to claim 4, **characterised in that** in each case two transmission levers (38, 39) in a common rotation point (39) are connected to the inner shank (10), at the sides of the inner shank (10) opposite to one another.

6. A medical forceps according to one of the preceding claims, **characterised in that** at the distal end (14) of the inner shank (10) there is formed at least one radially outwardly projecting projection (34) which engages into a corresponding recess (32) at the distal end of the outer shank (8), wherein the recess (32) in the longitudinal direction of the outer (8) and of the inner shank (10) has a greater extension than the projection (34).

7. A medical forceps according to claim 6, **characterised in that** on the inner shank (10) there is formed in each case one projection (34) on each of the two sides opposite one another, and the outer shank (8) comprises two corresponding recesses (32) at the sides opposite one another, into which the projections (34) engage.

8. A medical forceps according to one of the preceding claims, **characterised in that** the linkage levers (44, 46) of the two jaw parts (26, 28) on each of the two sides opposite one another are designed in a manner such that the linkage lever (44) of the first jaw part (26) is designed lying radially further inwards than the linkage lever (46) of the second jaw part (28).

9. A medical forceps according to one of the claims 3 to 7, **characterised in that** the two transmission levers (38, 39) on each of the two sides opposite one another in each case are arranged lying radially over one another.

10. A medical forceps according to one of the preceding claims, **characterised in that** the linkage levers (44, 46) of the two jaw parts (26, 28) on each of the two sides opposite one another are designed in a manner such that seen in the longitudinal direction of the outer shank (8) in the closed condition of the forceps jaw (4), the free end of the linkage lever (44) of the first jaw part (26) extends in the extension of the second jaw part (28) and the free end of the linkage lever (46) of the second jaw part (28) extends in the extension of the first jaw part (26).

11. A medical forceps according to one of the preceding claims, **characterised in that** a hole (52) is formed in at least one of the two jaw parts (26, 28).

## Revendications

1. Pince à usage médical comprenant
une tige extérieure tubulaire (8), à l'extrémité distale de laquelle est formée une mâchoire de pince (4) comportant deux éléments de mâchoire (26, 28), les deux éléments de mâchoire (26, 28) étant montés à rotation sur la tige extérieure (8) respectivement sur deux côtés opposés l'un à l'autre,
une tige intérieure tubulaire (10) destinée à actionner les éléments de mâchoire (26, 28), laquelle est disposée à coulissement libre dans sa direction longitudinale (x) à l'intérieur de la tige extérieure (8), la tige intérieure (10) étant accouplée, par l'intermédiaire de deux systèmes de leviers (38, 39), aux deux éléments de mâchoire (26, 28), en vue de leur actionnement, **caractérisée en ce que**
les deux éléments de mâchoire (26, 28) présentent des leviers d'articulation (44, 46) respectifs s'étendant vers le côté proximal, dans la direction longitudinale (x) de la tige extérieure (8) et de la tige intérieure (10), respectivement sur chacun des deux côtés opposés l'un à l'autre, lesquels leviers d'articulation sont accouplés à la tige intérieure (10), chacun par l'intermédiaire de l'un des deux systèmes de leviers (38, 39), les leviers d'articulation (44, 46) et le système de leviers (38, 39) étant disposés de façon à être positionnés plus loin vers l'extérieur, en direction radiale, que la surface périphérique intérieure de la tige intérieure (10).

2. Pince à usage médical selon la revendication 1, **caractérisée en ce que** les deux éléments de mâchoire (26, 28) sont, sur la tige extérieure (8), montés en un centre de rotation commun (30), respectivement sur les deux côtés opposés l'un à l'autre.

3. Pince à usage médical selon la revendication 1 ou 2, **caractérisée en ce que** les leviers d'articulation (44, 46) sont chacun reliés par l'intermédiaire d'un levier de transmission (38, 39) respectif à la tige intérieure (10).

4. Pince à usage médical selon la revendication 3, **caractérisée en ce que** les leviers de transmission (38, 39) sont chacun reliés, au niveau d'une première extrémité (40, 42), en rotation à un levier d'articulation (44, 46) et, au niveau d'une deuxième extrémité (36), en rotation à la tige intérieure.

5. Pince à usage médical selon la revendication 4, **caractérisée en ce que** sur les côtés opposés l'un à l'autre de la tige intérieure (10), deux leviers de transmission (38, 39) sont respectivement reliés, en un centre de rotation commun (39), à la tige intérieure (10).

6. Pince à usage médical selon l'une des revendications précédentes, **caractérisée en ce qu'**à l'extrémité distale (14) de la tige intérieure (10), est formée au moins une saillie (34) s'avançant vers l'extérieur en direction radiale, laquelle saillie s'engage dans un évidement (32) correspondant, à l'extrémité distale de la tige extérieure (8), l'évidement (32) présentant, dans la direction longitudinale de la tige extérieure (8) et de la tige intérieure (10), une dimension plus grande que la saillie (34).

7. Pince à usage médical selon la revendication 6, **caractérisée en ce que** sur la tige intérieure (10), une saillie (34) est respectivement formée sur chacun des deux côtés opposés l'un à l'autre, et la tige extérieure (8) présente, sur les côtés opposés l'un à l'autre, deux évidements (32) correspondants, dans lesquels les saillies (34) s'engagent.

8. Pince à usage médical selon l'une des revendications précédentes, **caractérisée en ce que** les leviers d'articulation (44, 46) des deux éléments de mâchoire (26, 28) sont, sur chacun des deux côtés opposés l'un à l'autre, réalisés d'une manière telle, que le levier d'articulation (44) du premier élément de mâchoire (26) soit positionné davantage à l'intérieur, en direction radiale, que le levier d'articulation (46) du deuxième élément de mâchoire (28).

9. Pince à usage médical selon l'une des revendications 3 à 7, **caractérisée en ce que** les deux leviers de transmission (38, 39) sont, sur chacun des deux côtés opposés l'un à l'autre, respectivement disposés de façon à être placés l'un sur l'autre en direction radiale.

10. Pince à usage médical selon l'une des revendications précédentes, **caractérisée en ce que** les leviers d'articulation (44, 46) des deux éléments de mâchoire (26, 28) sont, sur chacun des deux côtés opposés l'un à l'autre, réalisés d'une manière telle, que considérées dans l'état fermé de la mâchoire de pince (4), l'extrémité libre du levier d'articulation (44) du premier élément de mâchoire (26) s'étende dans le prolongement du deuxième élément de mâchoire (28) et l'extrémité libre du levier d'articulation (46) du deuxième élément de mâchoire (28) s'étende dans le prolongement du premier élément de mâchoire (26), dans la direction longitudinale de la tige extérieure (8).

11. Pince à usage médical selon l'une des revendications précédentes, **caractérisée en ce qu'**un trou (52) est ménagé dans au moins l'un des deux éléments de mâchoire (26, 28).
